# EUROPÄISCHE PATENTANMELDUNG

(11) **EP 4 151 204 A1**
(43) Veröffentlichungstag der Anmeldung: **22.03.2023**
(21) Anmeldenummer: 21197405.0
(22) Anmeldetag: 17.09.2021
(51) Int. Cl.: A61K 9/00, A61K 31/135, A61K 31/485, A61K 31/495, A61K 31/57, A61P 15/18, A61P 25/04, A61P 25/24, A61P 25/36

(54) **SCHNELL ZERFALLENDE ORALE DÜNNE FILME/SCHÄUME MIT HOHER WIRKSTOFFBELADUNG AUF BASIS EINES GEMISCHES VON POLYVINYLALKOHOLEN MIT UNTERSCHIEDLICHEN MOLEKULARGEWICHTEN**

(71) Anmelder: LTS Lohmann Therapie-Systeme AG, 56626 Andernach (DE)
(72) Erfinder: SEIBERTZ, Frank, 53498 Bad Breisig (DE); BAUER, Marius, 56626 Andernach (DE); FUHRMANN, Marlene, 56829 Kail (DE); LINN, Michael, 55596 Waldböckelheim (DE); EMGENBROICH, Marco, 53359 Rheinbach (DE)
(74) Vertreter: Held, Stephan

(57) **Zusammenfassung**

Beschrieben wird ein oraler dünner Film, umfassend mindestens eine Matrixschicht, wobei die mindestens eine Matrixschicht mindestens einen Polyvinylalkohol mit einem niedrigeren mittleren Molekulargewicht und mindestens einen Polyvinylalkohol mit einem höheren mittleren Molekulargewicht und mindestens einen pharmazeutischen Wirkstoff umfasst, und dessen Verwendung als Arzneimittel.

## Beschreibung

Die vorliegende Erfindung betrifft einen oralen dünnen Film, enthaltend mindestens einem pharmazeutischen Wirkstoff, und die Verwendung eines solchen oralen dünnen Films als Arzneimittel.

Orale dünne Filme (OTF = oral thin film), auch als orale Dünnfilme bezeichnet, sind dünne, flexible Filme, die auf einer Polymermatrix basieren und mit Wirkstoffen zur Medikamentenabgabe beladen sind. Die oralen dünnen Filme werden oral eingenommen und lösen sich sofort im Mund auf oder werden auf die Schleimhaut aufgetragen. Sie werden auf oder unter der Zunge bzw. bukkal platziert, wo sie sich dann auflösen oder zerfallen.

Orale dünne Filme können als nicht geschäumte Filme oder als geschäumte Filme bzw. Schaum ausgebildet sein. OTF werden manchmal auch als orale dünne Folien bezeichnet.

Die EP 422100 B1 offenbart den Wirkstoff Ulipristalacetat. Ulipristalacetat ist ein bekanntes Notfallkontrazeptivum ("Pille danach"). Sie wird als Tablette ("EllaOne^{®}") verabreicht, die 30 mg mikronisiertes Ulipristalacetat und als weitere Bestandteile Lactose-Monohydrat, Povidon, Croscarmellose-Natrium und Magnesiumstearat enthält. EllaOne^{®} wurde im Jahr 2009 in der Europäischen Union zugelassen.

Ketamin ist ein anderer bekannter pharmazeutischer Wirkstoff, der insbesondere der Behandlung oder Prävention von Schmerzen dient.

Bei der Verabreichung von manchen pharmazeutischen Wirkstoffen sind hohe Wirkstoffbeladungen des oralen dünnen Films wünschenswert, z.B. orale dünne Filme mit Ulipristalacetat als Wirkstoff mit 30 mg pro Einzeldosis. Bei solchen oralen dünnen Filmen bzw. oralen Schäumen mit hoher Wirkstoffbeladung pro Einzeldosis besteht aber das Problem, dass vergleichsweise große Mengen an Polymer benötigt werden, um einen Film bzw. Schaum herzustellen, der eine akzeptable Flexibilität besitzt, so dass brüchige Filme vermieden werden. Die hohen Polymeranteile führen aber dazu, dass sich die Dicke bzw. das Flächengewicht der Filme oder Schäume erhöhen. In der Folge erhöht sich bei diesen Formulierungen auch die Zerfallszeit. Dies ist für Filme bzw. Schäume unerwünscht, die sehr schnell im Mund zerfallen sollen.

In ersten getesteten Schaumformulierungen mit Ulipristalacetat als Wirkstoff auf Basis von Polyvinylalkohol (PVA) mit niedrigem Molekulargewicht (PVA 4-88, MW ca. 31,000 Da) betrug der Anteil des PVA 63,9 %. Mit dieser Konzentration konnte ein Ulipristalacetat-Schaum mit guten haptischen Eigenschaften hergestellt werden (siehe Formulierung A1 im experimentellen Teil). Die hohe Polymerkonzentration führte aber, wie oben beschrieben, zu einer Erhöhung des Flächengewichts (180 g/m²) und der Zerfallszeit.

Zudem haben bekannte orale dünne Filme mit hoher Wirkstoffbeladung den Nachteil, dass das maximale Flächengewicht und damit die Menge an enthaltenem pharmazeutisch aktivem Wirkstoff durch die Trocknung des oralen dünnen Films bei dessen Herstellung bestimmt wird. Je größer das Flächengewicht des oralen dünnen Films ist, desto mehr pharmazeutisch aktiver Wirkstoff kann zwar darin enthalten sein, jedoch wird dadurch die Trockenzeit des oralen dünnen Films auf eine nicht mehr wirtschaftliche Zeit verlängert und es kann zudem zu einer inhomogenen Verteilung des Wirkstoffs in dem oralen dünnen Film kommen.

Die Aufgabe der vorliegenden Erfindung besteht darin, vorstehend genannte Nachteile zu beheben. Insbesondere besteht die Aufgabe der vorliegenden Erfindung darin, flexible und stabile orale dünne Filme/Schäume bereitzustellen, die eine hohe Wirkstoffbeladung ermöglichen und dabei gleichzeitig eine akzeptable Filmdicke und eine schnelle Zerfallszeit bei Anwendung aufweisen.

Diese Aufgabe konnte gemäß Anspruch 1 überraschenderweise durch den Einsatz eines Gemisches aus Polyvinylalkoholen mit unterschiedlichen Molekulargewichten in der Matrixschicht erreicht werden. Eine zusätzliche Verbesserung konnte durch Zugabe von Feuchthaltemitteln, insbesondere bestimmtem Zuckeralkoholen oder Stärkederivaten, erreicht werden.

Die Erfindung betrifft dementsprechend einen oralen dünnen Film, umfassend mindestens eine Matrixschicht, wobei die mindestens eine Matrixschicht mindestens einen Polyvinylalkohol mit einem niedrigeren mittleren Molekulargewicht von 10.000 bis etwa 75.000 g/mol, bevorzugt 10.000 bis etwa 40.000 g/mol, und mindestens einen Polyvinylalkohol mit einem höheren mittleren Molekulargewicht von 140.000 bis 300.000 g/mol, bevorzugt 140.000 bis 220.000 g/mol und mindestens einen pharmazeutischen Wirkstoff umfasst, wobei der mindestens eine Polyvinylalkohol mit einem niedrigeren mittleren Molekulargewicht und der mindestens eine Polyvinylalkohol mit einem höheren mittleren Molekulargewicht in einer Gesamtmenge von 15 bis 70 Gew.-%, vorzugsweise von 15 bis 65 Gew.-%, bezogen auf das Gesamtgewicht der Matrixschicht, in der Matrixschicht vorhanden sind und das Gewichtsverhältnis von dem mindestens einen Polyvinylalkohol mit einem niedrigeren mittleren Molekulargewicht zu dem mindestens einen Polyvinylalkohol mit einem höheren mittleren Molekulargewicht im Bereich von 20:1 bis 2:1 liegt.

Der nebengeordnete unabhängige Anspruch betrifft die Verwendung des erfindungsgemäßen oralen dünnen Films. Bevorzugte Ausführungsformen sind in den abhängigen Ansprüchen angegeben.

Im Folgenden wird PVA als Abkürzung für Polyvinylalkohol verwendet. Der erfindungsgemäß eingesetzte Polyvinylalkohol mit einem niedrigeren mittleren Molekulargewicht von 10.000 bis etwa 75.000 g/mol, bevorzugt 10.000 bis etwa 40.000 g/mol wird im Folgenden als auch als "niedrigmolekularer Polyvinylalkohol" bzw. "niedrigmolekularer PVA" bezeichnet. Der Polyvinylalkohol mit einem höheren mittleren Molekulargewicht von 140.000 bis 300.000 g/mol, bevorzugt 140.000 bis 220.000 g/mol wird im Folgenden als auch als "hochmolekularer Polyvinylalkohol" bzw. "hochmolekularer PVA" bezeichnet. In der vorliegenden Schrift kann "umfassend" auch "bestehend aus" bedeuten.

Durch die Zugabe einer relativ niedrigen Konzentration, z.B. 6 Gew.-%, an hochmolekularem PVA, z.B. PVA 40-88, zu einem niedrigmolekularen PVA war es überraschenderweise möglich, die Gesamtpolymerkonzentration in der Formulierung deutlich zu reduzieren (im Beispiel von 65,8% (Formulierung A1) auf 39 % (Formulierung A2)), ohne dabei die Formstabilität der vorherigen Formulierungen zu verlieren und die Zerfallszeit merklich zu erhöhen. Da die erfindungsgemäße PVA-Mischung, z.B. PVA 4-88/40-88, den Einsatz einer geringeren Polymergesamtkonzentration zulässt, konnte das Flächengewicht der oralen Filme ebenfalls deutlich reduziert werden, z.B. von 180 auf 109,5 g/m². Hierdurch konnte im Vergleich zu Formulierungen, die nur niedrigmolekularen PVA enthalten, die Zerfallszeit verbessert werden.

Ohne sich an eine Theorie binden zu wollen, wird davon ausgegangen, dass das längerkettige PVA wie PVA 40-88 in diesem Gemisch eine Art Strukturbildner darstellt, der die Einarbeitung geringerer PVA Konzentrationen ermöglicht.

Der erfindungsgemäße orale dünne Film weist mindestens eine Matrixschicht auf. Die mindestens eine Matrixschicht umfasst mindestens einen Polyvinylalkohol mit einem niedrigeren mittleren Molekulargewicht im Bereich von 10.000 bis etwa 75.000 g/mol, bevorzugt 10.000 bis 40.000 g/mol, bevorzugter 25.000 bis 35.000 g/mol, und mindestens einen Polyvinylalkohol mit einem höheren mittleren Molekulargewicht im Bereich von 140.000 bis 300.000 g/mol, bevorzugt 140.000 bis 220.000 g/mol, bevorzugter 200.000 bis 210.000 g/mol.

Sofern nicht anders angegeben, beziehen sich alle genannten mittleren Molekulargewichte von Polymeren auf das gewichtsgemittelte Molekulargewicht (Mw), das mittels Gelpermeationschromatographie bestimmt wird.

Zur Herstellung von PVA wird in der Regel zunächst Polyvinylacetat hergestellt, dessen Acetatgruppen dann hydrolysiert werden. Handelsübliche Polyvinylalkohole können Hydrolysegrade von 84 bis 92 Mol-%, vorzugsweise 86 bis 90 Mol-%, aufweisen, enthalten also noch einen Restgehalt an Acetyl-Gruppen, häufig sind Hydrolysegrade von 98 bis 99 bzw. 86 bis 89 Mol-%.

Gemäß der vorliegenden Erfindung ist eine Kombination von mindestens einem Polyvinylalkohol mit einem mittleren Molekulargewicht von etwa 31.000 g/mol und mindestens einem Polyvinylalkohol mit einem mittleren Molekulargewicht von etwa 205.000 g/mol besonders geeignet.

In einer bevorzugten Ausführungsform umfasst der orale dünne Film daher eine Matrixschicht, in der der mindestens eine Polyvinylalkohol mit einem niedrigeren mittleren Molekulargewicht Polyvinylalkohol 4-88 oder Polyvinylalkohol 8-88 ist und/oder der mindestens eine Polyvinylalkohol mit einem höheren mittleren Molekulargewicht Polyvinylalkohol 40-88 ist. Diese Polyvinylalkohole sind im Handel erhältlich. Bei einem zu hohen Molekulargewicht des Polyvinylalkohols mit einem höheren mittleren Molekulargewicht ergibt sich eine zu hohe Viskosität der erhaltenen Mischung, wodurch eine Verarbeitung erschwert bzw. kaum möglich wird. Bei einem zu niedrigen Molekulargewicht ergibt sich keine ausreichende Strukturierung der erhaltenen Mischung.

Die Gesamtmenge des mindestens einen Polyvinylalkohol mit einem niedrigeren mittleren Molekulargewicht und des mindestens einen Polyvinylalkohol mit einem höheren mittleren Molekulargewicht in der Matrixschicht liegt im Bereich von 15 bis 65 Gew.-%, bevorzugt 20 bis 60 Gew.-%, bevorzugter 22 bis 58 Gew.-%, insbesondere 24 bis 56 Gew.-% oder von 22 bis 50 Gew.-% oder von 25 bis 40 Gew.-%, bezogen auf das Gesamtgewicht der Matrixschicht.

Das Gewichtsverhältnis (A:B) von dem mindestens einen Polyvinylalkohol mit einem niedrigeren mittleren Molekulargewicht (A) zu dem mindestens einen Polyvinylalkohol mit einem höheren mittleren Molekulargewicht (B) liegt im Bereich von 20:1 bis 2:1, bevorzugt von 15:1 bis 3:1, bevorzugter 12:1 bis 4:1, oder von 10:1 bis 4:1 oder von 10:1 bis 2:1, insbesondere von 9:1 bis 7:1.

Die Menge des mindestens einen Polyvinylalkohol mit einem höheren mittleren Molekulargewicht in der Matrixschicht kann dabei relativ klein sein, z.B. im Bereich von 1 bis 18 Gew.-% oder von 1 bis 16 Gew.-% oder von 1 bis 12 Gew.-%, bevorzugt 2 bis 10 Gew.-%, insbesondere 3 bis 4 Gew.-%, bezogen auf das Gesamtgewicht der Matrixschicht.

Es ist bevorzugt, dass der mindestens eine Polyvinylalkohol mit einem niedrigeren mittleren Molekulargewicht ein Polyvinylalkohol mit einem niedrigeren mittleren Molekulargewicht ist und der mindestens eine Polyvinylalkohol mit einem höheren mittleren Molekulargewicht ein Polyvinylalkohol mit einem höheren mittleren Molekulargewicht ist.

Die eingesetzten Polyvinylalkohole dienen als Matrixbildner für die Matrixschicht, die eine Polymermatrix umfasst. Es ist bevorzugt, dass in der Matrixschicht neben diesen Polyvinylalkoholen keine weiteren Polymere, insbesondere keine weiteren matrixbildenden Polymere, enthalten sind. Sofern weitere Polymere enthalten sind, ist die Menge an Polymeren, die von den genannten Polyvinylalkoholen verschieden sind, bevorzugt nicht mehr als 15 Gew.-%, bevorzugter nicht mehr als 10 Gew.-%, bezogen auf das Gesamtgewicht der Matrixschicht. Beispiele für weiterer Polymere, die gegebenenfalls eingesetzt werden können, sind z.B. Hydroxypropylmethylcellulose (HPMC), Polyvinylpyrrolidon (PVP), Hydroxypropylcellulose (HPC), Polyethylenglycol (PEG) oder Mischungen dieser Polymere.

Die Matrixschicht des erfindungsgemäßen oralen dünnen Films umfasst ferner mindestens einen pharmazeutischen Wirkstoff, z.B. einen pharmazeutischen Wirkstoff oder eine Kombination aus zwei oder mehr pharmazeutischen Wirkstoffen. Der mindestens eine pharmazeutisch aktive Wirkstoff unterliegt prinzipiell keiner Beschränkung, ist aber vorzugsweise ausgewählt aus allen pharmazeutischen Wirkstoffen, die oral und/oder transmukosal verabreicht werden können. Gemäß der vorliegenden Erfindung werden unter dem pharmazeutischen Wirkstoff z.B. auch alle pharmazeutisch akzeptablen Salze und Solvate des jeweiligen pharmazeutischen Wirkstoffs subsumiert.

Der mindestens eine pharmazeutische Wirkstoff kann beispielsweise ausgewählt sein aus pharmazeutischen Wirkstoffen der Wirkstoffklassen der Notfallkontrazeptiva, Analgetika, Hormone, Hypnotika, Sedativa, Antiepileptika, Weckamine, Psychoneurotropika, Neuro-Muskelblocker, Antispasmodika, Antihistaminika, Antiallergika, Cardiotonika, Antiarrhythmika, Diuretika, Hypotensiva, Vasopressoren, Antidepressiva, Antitussiva, Expectorantia, Thyroidhormone, Sexualhormone, Antidiabetika, Antitumor-Wirkstoffe, Antibiotika, Chemotherapeutika und Narkotika, wobei die Liste nicht abschließend ist.

Der mindestens eine pharmazeutische Wirkstoff ist bevorzugt ein partikulärer pharmazeutischer Wirkstoff, der in der Matrix bzw. Polymermatrix dispergiert ist. Der mindestens eine pharmazeutische Wirkstoff kann ein wasserlöslicher Wirkstoff oder ein in Wasser schwer löslicher oder wasserunlöslicher Wirkstoff sein. Hierin bezieht sich ein wasserlöslicher Wirkstoff auf einen Wirkstoff mit einer Löslichkeit in Wasser von mehr als 10 g/L bei einer Temperatur von 20 °C Hierin bezieht sich ein in Wasser schwer löslicher oder wasserunlöslicher Wirkstoff auf einen Wirkstoff mit einer Löslichkeit in Wasser von nicht mehr als 10,0 g/L, bei einer Temperatur von 20 °C. Die Angaben leiten sich von den Definitionen im Europäischen Arzneibuch ab.

Der Wirkstoff kann dabei in der Formulierung zur Herstellung des oralen dünnen Films in gelöster Form, in suspendierter Form oder sowohl in gelöster als auch in suspendierter Form vorliegen.

Der Wirkstoff kann als Ausgangsmaterial in Partikelform vorliegen. Wenn das Ausgangsmaterial des Wirkstoffs partikelförmig ist, kann es sich um mikronisierte Partikel oder nicht mikronisierte Partikel handeln. Die Partikelgröße kann z.B. im Bereich von 0,2 µm bis 1 mm liegen.

In einer bevorzugten Ausführungsform ist oder umfasst der mindestens eine pharmazeutische Wirkstoff Ulipristalacetat, bevorzugt mikronisiertes Ulipristalacetat. Ulipristalacetat ist 17-Acetoxy-11-(4-N,N-dimethylaminophenyl)-19-norpregna-4,9-dien-3,20-dion) mit der folgenden chemischen Formel:

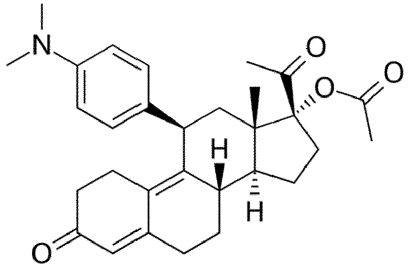

Der Wirkstoff Ulipristalacetat ist in der Polymermatrix der Matrixschicht dispergiert. In der Matrixschicht liegt Ulipristalacetat bevorzugt in fester Form, insbesondere in kristalliner Form, als Partikel vor.

Das Ulipristalacetat als Ausgangsmaterial kann z.B. nicht mikronisiertes oder mikronisiertes Ulipristalacetat sein. Die Partikelgröße des Ulipristalacetats kann dabei z.B. im Bereich von 0,2 µm bis 1mm, z.B. 0,2 bis 100,0 µm oder 0,5 bis 40,0 µm, liegen.

Die Partikelgröße bezieht sich auf die volumengewichtete Partikelgröße d90 und kann z. B. durch Laserbeugungsanalyse, dynamische Lichtstreuung oder Siebanalyse bestimmt werden. Die volumengewichtete Partikelgröße d90 bezieht sich auf die volumengewichtete Partikelgröße, bei der 90% der Verteilung eine kleinere Partikelgröße und zehn Prozent eine größere Partikelgröße aufweisen, wie dem Fachmann bekannt ist.

In einer weiteren bevorzugten Ausführungsform umfasst oder ist der mindestens eine pharmazeutische Wirkstoff Ketamin oder ein pharmazeutisch akzeptables Salz davon. Unter Ketamin wird hierbei (S)-(±)-2-(2-Chlorphenyl)-2-(methylamino)cyclohexan-1-on, (R)-(±)-2-(2-Chlorphenyl)-2-(methylamino)cyclohexan-1-on, sowie das Racemat (RS)-(±)-2-(2-Chlorphenyl)-2-(methylamino)cyclohexan-1-on verstanden. Das Ketamin ist vorzugsweise (S)-Ketamin oder ein pharmazeutisch akzeptables Salz davon, wobei Ketamin bevorzugt als HCl Salz, wie z.B. (S)-Ketamin HCl, oder als freie Base vorliegt. wie z.B. (S)-Ketamin HCl, vorliegt. Ketamin wird als Analgetikum, Antidepressivum oder Anästhetikum eingesetzt.

Das Ketamin als Ausgangsmaterial kann als mikronisierte Partikel oder als nicht mikronisierte Partikel vorliegen. Geeignete mittlere Partikelgrößen liegen z.B. im Bereich von 1 bis 1000 µm oder von 5 bis 500 µm oder von 10 bis 200 µm. Die Partikelgröße kann beispielsweise mittels Lichtmikroskop bestimmt werden.

In einer weiteren bevorzugten Ausführungsform umfasst oder ist der mindestens eine pharmazeutische Wirkstoff Naloxon, insbesondere Naloxon HCl Dihydrat. Naloxon ist ein Opioidantagonist und gehört zu den reinen Opioidantagonisten, die als kompetitive Antagonisten an allen Opioidrezeptoren wirken. Damit heben sie die Wirkungen, die durch Opiate und Opioide verursacht werden, teilweise oder ganz auf.

In einer weiteren bevorzugten Ausführungsform umfasst oder ist der mindestens eine pharmazeutische Wirkstoff Nalmefen, insbesondere Nalmefen HCl. Nalmefen ist ein Opioidantagonist und hebt damit die Wirkungen, die durch Opiate und Opioide verursacht werden, teilweise oder ganz auf. Ferner wird Nalmefen in er Behandlung der Alkoholkrankheit eingesetzt.

In einer weiteren bevorzugten Ausführungsform umfasst oder ist der mindestens eine pharmazeutische Wirkstoff Vortioxetin, insbesondere Vortioxetin HBr. Vortioxetin ist ein antidepressiv wirkender Arzneistoff mit multimodalem Wirkmechanismus.

Der mindestens eine pharmazeutische Wirkstoff, oder ein pharmazeutisch akzeptables Salz davon, ist bevorzugt in einer Menge im Bereich von 10 bis 60 Gew.-%, bevorzugt 20 bis 55 Gew.-%, bevorzugter 30 bis 50 Gew.-%, bezogen auf das Gesamtgewicht der Matrixschicht, in der Matrixschicht vorhanden.

In einer besonders bevorzugten Ausführungsform beinhaltet die Matrixschicht ferner mindestens ein hygroskopisches Additiv mit mehr als 3 Kohlenstoffatomen, welches bevorzugt ausgewählt ist aus Zuckern, Zuckeralkoholen, Stärke und Stärkederivaten oder Mischungen davon.

Die zusätzliche Aufnahme eines hygroskopischen Hilfsstoffes wie z.B. Zucker, Zuckeralkohole, Stärke und/oder Stärkederivate, führt zu einer weiteren merklichen Reduktion der Zerfallszeit (siehe Formulierung A2 in den Beispielen). Ohne sich an eine Theorie binden zu wollen, wird davon ausgegangen, dass der Effekt darauf beruht, dass die hygroskopischen Hilfsstoffe schnell Wasser in die Formulierung "ziehen", was dann aufgrund der niedrigen Polymerkonzentration zu einer schnelleren Zerfallszeit gegenüber einer Referenzformulierung ohne einen solchen hygroskopischen Hilfsstoff führt.

Bevorzugte konkrete Beispiele für geeignete hygroskopischen Hilfsstoffe mit mehr als 3 Kohlenstoffatomen sind Mannit (Mannitol), Isomalt, Lactit, Sorbit (Sorbitol oder Glucitol) und Xylit (Xylitol), Threit, Erythrit, Stärken wie Mais- oder Reisstärke, einschließlich Stärkederivaten, Sucralose oder Mischungen von zwei oder mehr dieser Verbindungen. Ein besonders bevorzugtes hygroskopisches Additiv ist Sorbitol. Die hygroskopischen Additive können hier auch als Zerfallsbeschleuniger bezeichnet werden.

Es kann ein hygroskopischer Hilfsstoff oder eine Kombination von zwei oder mehr aufgenommen werden. Der mindestens eine hygroskopische Hilfsstoff mit mehr als 3 Kohlenstoffatomen kann, falls eingesetzt, z.B. in einer Menge von bis zu 20 Gew.-%, bevorzugt in einer Menge von 5 bis 20 Gew.-%, bevorzugter 5 bis 15 Gew.-%, bezogen auf das Gesamtgewicht der Matrixschicht, in der Matrixschicht enthalten sein.

Die Matrixschicht kann ferner mindestens einen Hilfsstoff umfassen, z.B. ausgewählt aus der Gruppe, bestehend aus Farbstoffen, Aromastoffen, Süßstoffen, Weichmachern, geschmacksmaskierenden Mitteln, Emulgatoren, Enhancern, pH-Regulatoren, Sprengmitteln, Lösungsvermittlern, Konservierungsmitteln, AntiSchaummittel (nur für die Filmherstellung) und/oder Antioxidationsmitteln. Es ist bevorzugt, dass die Matrixschicht einen oder mehrere Weichmacher, vorzugsweise Glycerin, umfasst.

Jeder dieser Hilfsstoffe ist, sofern enthalten, vorzugsweise jeweils in einer Menge von 0,1 bis 15 Gew.-%, bevorzugt von 0,1 bis 10 Gew.-% oder von 0,1 bis 5 Gew.-%, bezogen auf das Gesamtgewicht der Matrixschicht, in der Matrixschicht enthalten.

Die Matrixschicht des oralen dünnen Films kann eine nicht geschäumte Matrixschicht oder eine geschäumte Matrixschicht sein, wobei die Matrixschicht bevorzugt ein Schaum ist. Die Gestaltung als nicht geschäumte Matrixschicht oder als geschäumte Matrixschicht ist den Fachleuten auf dem Gebiet bekannt.

Bei einer geschäumten Matrixschicht kann die Schicht in Form eines verfestigten Schaums vorliegen, der Räume oder Hohlräume aufweist, die mit einem Gas, einem Gasgemisch, einer Flüssigkeit oder einem Flüssigkeitsgemisch gefüllt sind. Solche OTFs werden üblicherweise als "Schaum-OTFs" bezeichnet. Durch die Hohlräume in Schaum-OTFs und die damit verbundene größere Oberfläche der Filme kann die Auflösung der Darreichungsform und die Wirkstofffreisetzung beschleunigt werden.

Die Bestandteile der erfindungsgemäßen Matrixschicht ermöglichen neben der Herstellung von Schäumen auch die Herstellung von nicht geschäumten OTFs mit hoher Wirkstoffbeladung (z.B. 43%) und akzeptabler Zerfallszeit (siehe Beispiel der Formulierung A4). Ohne den Zusatz des hochmolekularen PVA müsste man die maximale Wirkstoffbeladung niedriger wählen, was durch eine Erhöhung des Flächengewichts kompensiert werden müsste. Dies würde wiederum zu längeren Zerfallszeiten führen. Bei den nicht geschäumten OTFs kommt im Gegensatz zu den Schäumen noch erschwerend hinzu, dass die mögliche Flächengewichtserhöhung stark begrenzt ist. Erreicht man ein Flächengewicht von mehr als 170 g/m² wird der Trocknungsprozess sehr zeitaufwendig und somit unwirtschaftlich.

Das Flächengewicht der Matrixschicht liegt bevorzugt im Bereich von 50 bis 220 g/m², bevorzugter 50 bis 150 g/m², besonders bevorzugt von 70 bis 130 g/m².

Die Matrixschicht des erfindungsgemäßen oralen dünnen Films weisen z.B. eine Fläche im Bereich von 0,3 bis 20 cm², vorzugsweise 1 bis 10 cm², auf. Die Dicke der Matrixschicht kann z.B. im Bereich von 10 bis 3000 µm, bevorzugt von 90 µm bis 2000 µm, bevorzugter 40 bis 400 µm, liegen.

In einer bevorzugten Ausführungsform umfasst die Matrixschicht mindestens 1 mg, bevorzugt mindestens 20 mg, bevorzugter mindestens 30 mg Wirkstoff, z.B. 25 bis 150 mg, bevorzugt 30 bis 150 mg, Wirkstoff, insbesondere Ulipristalacetat, Ketamin, Naloxon, Nalmefen oder Vortioxetin, insbesondere Naloxon HCl Dihydrat oder Nalmefen HCL oder Vortioxetin HBr.

Besonders bevorzugt wird das Gewichtsverhältnis von dem mindestens einen Polyvinylalkohol mit einem niedrigeren mittleren Molekulargewicht zu dem mindestens einen Polyvinylalkohol mit einem höheren mittleren Molekulargewicht sowie die jeweiligen Gesamtmengen an dem mindestens einen Polyvinylalkohol mit einem niedrigeren mittleren Molekulargewicht und dem mindestens einen Polyvinylalkohol mit einem höheren mittleren Molekulargewicht an einen in dem oralen Dünnfilm enthaltenen Wirkstoff angepasst.

So ist es bevorzugt, dass in einem oralen Dünnfilm, insbesondere wie vorstehend definiert, in dem Ulipristalacetat oder ein pharmazeutisch akzeptables Salz davon als pharmazeutisch aktiver Wirkstoff enthalten ist, das Gewichtsverhältnis von dem mindestens einen Polyvinylalkohol mit einem niedrigeren mittleren Molekulargewicht zu dem mindestens einen Polyvinylalkohol mit einem höheren mittleren Molekulargewicht im Bereich von 10:1 bis 4:1 liegt. Zusätzlich oder unabhängig davon ist es bevorzugt, dass die Menge an dem mindestens einen Polyvinylalkohol mit einem niedrigeren mittleren Molekulargewicht im Bereich von 20 bis 40 Gew.-%, bezogen auf das Gesamtgewicht der Matrixschicht, liegt. Zusätzlich oder unabhängig davon ist es bevorzugt, dass die Menge an dem mindestens einen Polyvinylalkohol mit einem höheren mittleren Molekulargewicht 2 bis 10 Gew.-%, bezogen auf das Gesamtgewicht der Matrixschicht, beträgt. Zusätzlich oder unabhängig davon ist es bevorzugt, dass die Menge an Ulipristalacetat oder dem pharmazeutisch akzeptablen Salz davon 35 bis 45 Gew.-%, bezogen auf das Gesamtgewicht der Matrixschicht, beträgt.

Ferner ist es bevorzugt, dass in einem oralen Dünnfilm, insbesondere wie vorstehend definiert, in dem Ketamin oder ein pharmazeutisch akzeptables Salz davon als pharmazeutisch aktiver Wirkstoff enthalten ist, das Gewichtsverhältnis von dem mindestens einen Polyvinylalkohol mit einem niedrigeren mittleren Molekulargewicht zu dem mindestens einen Polyvinylalkohol mit einem höheren mittleren Molekulargewicht im Bereich von 10:1 bis 4:1 liegt. Zusätzlich oder unabhängig davon ist es bevorzugt, dass die Menge an dem mindestens einen Polyvinylalkohol mit einem niedrigeren mittleren Molekulargewicht im Bereich von 20 bis 40 Gew.-%, vorzugsweise von 25 bis 35 Gew.-%, bezogen auf das Gesamtgewicht der Matrixschicht, liegt. Zusätzlich oder unabhängig davon ist es bevorzugt, dass die Menge an dem mindestens einen Polyvinylalkohol mit einem höheren mittleren Molekulargewicht 2 bis 10 Gew.-%, bezogen auf das Gesamtgewicht der Matrixschicht, beträgt. Zusätzlich oder unabhängig davon ist es bevorzugt, dass die Menge an Ketamin oder dem pharmazeutisch akzeptablen Salz davon 35 bis 45 Gew.-%, bezogen auf das Gesamtgewicht der Matrixschicht, beträgt.

Ferner ist es bevorzugt, dass in einem oralen Dünnfilm, insbesondere wie vorstehend definiert, in dem Naloxon oder ein pharmazeutisch akzeptables Salz davon als pharmazeutisch aktiver Wirkstoff enthalten ist, das Gewichtsverhältnis von dem mindestens einen Polyvinylalkohol mit einem niedrigeren mittleren Molekulargewicht zu dem mindestens einen Polyvinylalkohol mit einem höheren mittleren Molekulargewicht im Bereich von 10:1 bis 2:1 liegt. Zusätzlich oder unabhängig davon ist es bevorzugt, dass die Menge an dem mindestens einen Polyvinylalkohol mit einem niedrigeren mittleren Molekulargewicht im Bereich von 45 bis 60 Gew.-%, vorzugsweise von 50 bis 58 Gew.-%, bezogen auf das Gesamtgewicht der Matrixschicht, liegt. Zusätzlich oder unabhängig davon ist es bevorzugt, dass die Menge an dem mindestens einen Polyvinylalkohol mit einem höheren mittleren Molekulargewicht 5 bis 20 Gew.-%, bezogen auf das Gesamtgewicht der Matrixschicht, beträgt. Zusätzlich oder unabhängig davon ist es bevorzugt, dass die Menge an Naloxon oder dem pharmazeutisch akzeptablen Salz davon 5 bis 15 Gew.-%, vorzugsweise etwa 10 Gew.-%, bezogen auf das Gesamtgewicht der Matrixschicht, beträgt.

Ferner ist es bevorzugt, dass in einem oralen Dünnfilm, insbesondere wie vorstehend definiert, in dem Nalmefen oder ein pharmazeutisch akzeptables Salz davon als pharmazeutisch aktiver Wirkstoff enthalten ist, das Gewichtsverhältnis von dem mindestens einen Polyvinylalkohol mit einem niedrigeren mittleren Molekulargewicht zu dem mindestens einen Polyvinylalkohol mit einem höheren mittleren Molekulargewicht im Bereich von 10:1 bis 2:1 liegt. Zusätzlich oder unabhängig davon ist es bevorzugt, dass die Menge an dem mindestens einen Polyvinylalkohol mit einem niedrigeren mittleren Molekulargewicht im Bereich von 45 bis 60 Gew.-%, vorzugsweise von 50 bis 58 Gew.-%, bezogen auf das Gesamtgewicht der Matrixschicht, liegt. Zusätzlich oder unabhängig davon ist es bevorzugt, dass die Menge an dem mindestens einen Polyvinylalkohol mit einem höheren mittleren Molekulargewicht 5 bis 20 Gew.-%, bezogen auf das Gesamtgewicht der Matrixschicht, beträgt. Zusätzlich oder unabhängig davon ist es bevorzugt, dass die Menge an Nalmefen oder dem pharmazeutisch akzeptablen Salz davon 5 bis 15 Gew.-%, vorzugsweise etwa 10 Gew.-%, bezogen auf das Gesamtgewicht der Matrixschicht, beträgt.

Ferner ist es bevorzugt, dass in einem oralen Dünnfilm, insbesondere wie vorstehend definiert, in dem Vortioxetin oder ein pharmazeutisch akzeptables Salz davon als pharmazeutisch aktiver Wirkstoff enthalten ist, das Gewichtsverhältnis von dem mindestens einen Polyvinylalkohol mit einem niedrigeren mittleren Molekulargewicht zu dem mindestens einen Polyvinylalkohol mit einem höheren mittleren Molekulargewicht von im Bereich 10:1 bis 2:1 liegt. Zusätzlich oder unabhängig davon ist es bevorzugt, dass die Menge an dem mindestens einen Polyvinylalkohol mit einem niedrigeren mittleren Molekulargewicht im Bereich von 20 bis 40 Gew.-%, vorzugsweise von 30 bis 40 Gew.-%, bezogen auf das Gesamtgewicht der Matrixschicht, liegt. Zusätzlich oder unabhängig davon ist es bevorzugt, dass die Menge an dem mindestens einen Polyvinylalkohol mit einem höheren mittleren Molekulargewicht 2 bis 12 Gew.-%, bezogen auf das Gesamtgewicht der Matrixschicht, beträgt. Zusätzlich oder unabhängig davon ist es bevorzugt, dass die Menge an Vortioxetin oder dem pharmazeutisch akzeptablen Salz davon 25 bis 35 Gew.-%, bezogen auf das Gesamtgewicht der Matrixschicht, beträgt.

Der erfindungsgemäße orale dünne Film ist bezüglich seines Aufbaus keinen Beschränkungen unterworfen. Die folgenden Gestaltungen sind unabhängig davon, ob die Matrixschicht geschäumt oder nicht geschäumt ist.

Der erfindungsgemäße OTF kann als einschichtiger oder mehrschichtiger Film ausgebildet sein, wobei ein einschichtiger Film bevorzugt ist. So kann der erfindungsgemäße orale dünne Film als einschichtiger oraler dünner Film vorliegen und damit lediglich aus der Matrixschicht, wie vorstehend definiert, bestehen.

In einer anderen Ausführungsform kann der OTF als mehrschichtiger OTF vorliegen und damit neben der Matrixschicht, wie vorstehend definiert, weitere Schichten enthalten. Diese zusätzlichen Schichten können direkt aufeinander laminiert werden oder aufeinander beschichtet werden oder mit einer dazwischenliegenden Klebeschicht verbunden werden. Unter Klebeschicht wird eine Schicht verstanden, die als Klebstoff, wie in der DIN EN 923:2016-03 definiert, wirken kann. Eine nichtklebende Schicht kann demnach nicht als Klebstoff wie vorstehend definiert wirken.

Als weitere Schichten können beispielsweise Pufferschichten zur Einstellung eines pH-Werts oder langsam lösliche oder unlösliche Schichten vorliegen, die den oralen dünnen Film vor vorzeitiger Erosion schützen. Alternativ können weitere Matrixschichten vorliegen, die andere pharmazeutisch aktive Wirkstoffe oder Geschmacksstoffe bzw. geschmacksmaskierende Stoffe enthalten. Weitere denkbare Schichten sind Haftschichten und schützende Deckschichten.

Der erfindungsgemäße orale dünne Film kann mit üblichen Verfahren hergestellt werden, die den Fachleuten bekannt sind. Übliche Verfahren zur Herstellung des erfindungsgemäßen oralen dünnen Films umfassen z.B. die Schritte:
a) Herstellen einer Lösung, Dispersion oder Schmelze, umfassend die Bestandteile der erfindungsgemäßen Matrixschicht, z.B. eine Dispersion in Wasser oder in einer Mischung von Wasser und Alkohol;
a1) optional Aufschäumen der Lösung, Dispersion oder Schmelze aus Schritt a) durch Eintragen eines Gases oder Gasgemisches, durch chemische Gaserzeugung oder durch Entspannen eines gelösten Gases oder mit Hilfe einer Schaumaufschlagmaschine;
b) Ausstreichen oder Gießen der Lösung, Dispersion oder Schmelze aus Schritt a) bzw. der optional aufgeschäumten Lösung, Dispersion oder Schmelze aus Schritt a1) auf eine Fläche oder in eine Form und Abkühlen oder Trocknen.

Die vorliegende Erfindung betrifft ferner einen oralen dünnen Film, wie vorstehend beschrieben, zur Verwendung als Arzneimittel.

In einer bevorzugten Ausführungsform wird der orale dünne Film als Notfallkontrazeptivum verwendet. In diesem Fall enthält der OTF ein Notfallkontrazeptivum als pharmazeutischen Wirkstoff, bevorzugt Ulipristalacetat. Die vorstehenden Angaben zu bevorzugten Ausführungsformen von Ulipristalacetat gelten selbstredend auch für die Verwendung.

In einer weiteren bevorzugten Ausführungsform wird der orale dünne Film zur Behandlung oder Prävention von Schmerzen und/oder Depressionen oder als Anästhetikum eingesetzt. In diesem Fall enthält der OTF insbesondere ein Analgetikum, ein Anästhetikum oder ein Antidepressivum als pharmazeutischen Wirkstoff, bevorzugt Ketamin oder Vortioxetin. Die vorstehenden Angaben zu bevorzugten Ausführungsformen von Ketamin oder Vortioxetin gelten selbstredend auch für die Verwendung.

In einer weiteren bevorzugten Ausführungsform wird der orale dünne Film in der Behandlung von Opiatüberdosierung eingesetzt. In diesem Fall enthält der OTF insbesondere eine Opioidantagonisten, als pharmazeutischen Wirkstoff, bevorzugt Naloxon, insbesondere Naloxon HCl Dihydrat, oder Nalmefen, insbesondere Nalmefen HCl.

Die Erfindung wird nachfolgend anhand von nicht beschränkenden Beispielen näher erläutert.

### Beispiele

### Beispiel 1:

Für alle der folgenden Formulierungen in Beispiel 1 gilt, dass die aus ihnen resultierenden einzeldosierten Arzneiformen eine Fläche von 7,04 cm² besitzen sollen und für die erfindungsgemäßen Beispiele eine Einzeldosis von mindestens 30 mg Ulipristalacetat bzw. Ketamin beinhalten sollen.

**Formulierung A1: Beispiel einer PVA Schaumformulierung ohne PVA Mix und Zuckeralkohole (Referenzformulierung)**

| Inhaltsstoff | Anteil in [Gew.-%] | Funktion des Inhaltsstoffs |
|---|---|---|
| Ulipristalacetat | 23,7 | Arzneistoff |
| PVA 4-88 | 64,2 | Filmpolymer |
| Glycerol | 8,8 | Weichmacher |
| Saccharin Na | 2,2 | Süßstoff |
| Sucralose | 1,1 | Süßstoff, Zerfallsbeschleuniger und Feuchhaltemittel |
| Flächengewicht | 180 g/m² | |
| Prozesslösemittel | Wasser | |

Da die Formulierung nur PVA 4-88 enthält, kann Sie nur mit einem hohen Polymeranteil von 64,2 % und daraus resultierend mit einem hohen Flächengewicht produziert werden. Wäre der Polymeranteil niedriger, wäre der Film zu brüchig und könnte im schlimmsten Fall nicht mehr konfektioniert werden. Das hohe Flächengewicht wirkt sich zudem negativ auf die Zerfallszeit aus.

**Formulierung A2: Beispiel einer PVA Schaumformulierung mit PVA Mix und Zuckeralkoholen**

| Inhaltsstoff | Anteil in [Gew.-%] | Funktion des Inhaltsstoffs |
|---|---|---|
| Ulipristalacetat | 39,0 | Arzneistoff |
| PVA 4-88 | 31,0 | Filmpolymer |
| PVA 40-88 | 6,0 | Filmpolymer |
| Xylitol | 2,0 | Süßstoff und Zerfallsbeschleuniger |
| Tween 80 | 1,0 | Weichmacher |
| Sorbitol | 9,0 | Süßstoff und Zerfallsbeschleuniger |
| Sucralose | 1,0 | Süßstoff, Zerfallsbeschleunigerund Feuchthaltemittel |
| Glycerol | 10,0 | Weichmacher |
| Menthol | 1 | Aroma |
| Flächengewicht | 109,5 g/m² | |
| Prozesslösemittel | Wasser | |

Diese Formulierung besitzt ein niedrigeres Flächengewicht als die Referenzformulierung. Zusätzlich besitzt sie akzeptable haptische Eigenschaften und eine wesentlich kürzere Zerfallszeit als die Referenz

**Formulierung A3: Beispiel einer PVA Schaumformulierung mit PVA Mix ohne Zuckeralkohole**

| Inhaltsstoff | Anteil in [Gew.-%] | Funktion des Inhaltsstoffs |
|---|---|---|
| Ulipristalacetat | 39,0 | Arzneistoff |
| PVA 4-88 | 38,0 | Filmpolymer |
| PVA 40-88 | 8,0 | Filmpolymer |
| Saccharin Na | 2,0 | Süßstoff |
| Tween 80 | 1,0 | Weichmacher |
| Sucralose | 1,0 | Süßstoff, Zerfallsbeschleuniger und Feuchthaltemittel |
| Glycerol | 10,0 | Weichmacher |
| Menthol | 1,0 | Aroma |
| Flächengewicht | 109,5 g/m² | |
| Prozesslösemittel | Wasser | |

Durch das Weglassen von Sorbitol verlängert sich die Zerfallszeit des Schaums (obwohl immer noch wesentlich schneller als die Referenz). Dies zeigt, dass Zuckeralkohole oder Stärkederivate in der Formulierung den Zerfall verbessern. Aufgrund der hohen Hydrophilie ziehen Zuckeralkohole oder Stärkederivate Wasser in den Schaum oder Film, wodurch die Löslichkeit verbessert wird.

**Formulierung A4: Beispiel einer PVA Filmformulierung mit PVA Mix, Zuckeralkoholen und Reisstärke**

| Inhaltsstoff | Anteil in [Gew.-%] | Funktion des Inhaltsstoffs |
|---|---|---|
| Ulipristalacetat | 43,0 | Arzneistoff |
| PVA 4-88 | 24,0 | Filmpolymer |
| PVA 40-88 | 3,0 | Filmpolymer |
| Span 85 | 1,0 | Weichmacher |
| Xylitol | 2,0 | Süßstoff und Zerfallsbeschleuniger |
| Tween 80 | 1,0 | Weichmacher |
| Sorbitol | 9,0 | Süßstoff und Zerfallsbeschleuniger |
| Sucralose | 1,0 | Süßstoff, Zerfallsbeschleuniger und Feuchthaltemittel |
| Glycerol | 9,0 | Weichmacher |
| Menthol | 1,0 | Aroma |
| Reisstärke | 6,0 | Zerfallsbeschleuniger |
| Flächengewicht | 99,5 g/m² | |
| Prozesslösemittel | Wasser | |

Der Film besitzt eine hohe Wirkstoffkonzentration und ein niedriges Flächengewicht. Obwohl es sich um einen Film handelt, besitzt die Formulierung eine kürzere Zerfallszeit als die Referenzformulierung.

**Formulierung B1: Beispiel einer PVA Filmformulierung mit PVA Mix und Zuckeralkoholen**

| Ausgangsstoffe | Zusammensetzung [Gew.-%] | Art / Funktion |
|---|---|---|
| Ketamin HCl | 38,0 | Wirkstoff |
| PVA 4-88 | 29,0 | Polymer |
| PVA 40-88 | 3,0 | Polymer |
| Span 85 | 1,0 | Weichmacher |
| Xylitol | 2,0 | Süßstoff und Zerfallsbeschleuniger |
| Tween 80 | 1,0 | Weichmacher |
| Sorbitol | 9,0 | Süßstoff und Zerfallsbeschleuniger |
| Sucralose | 1,0 | Süßstoff, Zerfallsbeschleuniger und Feuchthaltemittel |
| Glycerol | 9,0 | Weichmacher |
| Menthol | 1,0 | Aroma |
| Reisstärke | 6,0 | Zerfallsbeschleuniger |
| Flächengewicht | 112,5 g/m² | |
| Lösemittel | Wasser | |

**Formulierung B2: Beispiel einer PVA Schaumformulierung mit PVA Mix und Zuckeralkoholen**

| Ausgangsstoffe | Zusammensetzung [Gew.-%] | Art / Funktion |
|---|---|---|
| Ketamin HCl | 39,0 | Wirkstoff |
| PVA 4-88 | 31,0 | Polymer |
| PVA 40-88 | 6,0 | Polymer |
| Xylitol | 2,0 | Süßstoff und Zerfallsbeschleuniger |
| Tween 80 | 1,0 | Weichmacher |
| Sorbitol | 9,0 | Süßstoff und Zerfallsbeschleuniger |
| Sucralose | 1,0 | Süßstoff, Zerfallsbeschleuniger und Feuchthaltemittel |
| Glycerol | 10,0 | Weichmacher |
| Menthol | 1,0 | Aroma |
| Flächengewicht | 109,5 g/m² | |
| Lösemittel | Wasser | |

### Zerfallszeiten der oben genannten Formulierungen

Die Zerfallszeit wurde mit Methode Ph. Eur. 2.9.1. mit Gewicht und Platte bestimmt.

| Formulierung | Zerfallszeit Messung 1 | Zerfallszeit Messung 2 |
|---|---|---|
| A1 (Referenz) | 50 Sekunden | 51 Sekunden |
| A2 | 4 Sekunden | 4 Sekunden |
| A3 | 17 Sekunden | 18 Sekunden |
| A4 | 30 Sekunden | 31 Sekunden |
| B1 | 8 Sekunden | 8 Sekunden |
| B2 | 15 Sekunden | 15 Sekunden |

### Beispiel 2:

Es wurden orale Dünnfilme mit Naloxon HCl Dihydrat und Nalmefen HCL als Wirkstoff hergestellt und deren Zerfallszeiten nach Methode Ph. Eur. 2.9.1. mit Gewicht und Platte bestimmt.

**Formulierung C1: PVA-Schaumformulierung**

| Ausgangsstoffe | Zusammensetzung [Gew.-%] | Art / Funktion |
|---|---|---|
| Naloxon HCl Dihydrat | 10 | Wirkstoff |
| PVA 4-88 | 51 | Polymer |
| PVA 40-88 | 16 | Polymer |
| Xylitol | 2 | Süßstoff und Zerfallsbeschleuniger |
| Tween 80 | 1 | Weichmacher |
| Sorbitol | 9 | Süßstoff und Zerfallsbeschleuniger |
| Sucralose | 1 | Süßstoff, Zerfallsbeschleuniger und Feuchthaltemittel |
| Glycerol | 10 | Weichmacher |
| Flächengewicht | 128 g/m² | |
| Lösemittel | Wasser | |
| Zerfallszeit | 4 s | |

**Formulierung C2: PVA-Filmformulierung**

| Ausgangsstoffe | Zusammensetzung [Gew.-%] | Art / Funktion |
|---|---|---|
| Naloxon HCl Dihydrat | 10 | Wirkstoff |
| PVA 4-88 | 56 | Polymer |
| PVA 40-88 | 6 | Polymer |
| Xylitol | 2 | Süßstoff und Zerfallsbeschleuniger |
| Span 83 | 1 | Emulgator |
| Tween 80 | 1 | Weichmacher |
| Sorbitol | 9 | Süßstoff und Zerfallsbeschleuniger |
| Sucralose | 1 | Süßstoff, Zerfallsbeschleuniger und Feuchthaltemittel |
| Glycerol | 9 | Weichmacher |
| Reisstärke | 5 | Sprengmittel |
| Flächengewicht | 147 g/m² | |
| Lösemittel | Wasser | |
| Zerfallszeit | 33 s | |

**Formulierung C3: PVA-Schaumformulierung**

| Ausgangsstoffe | Zusammensetzung [Gew.-%] | Art / Funktion |
|---|---|---|
| Naloxon HCl Dihydrat | 10 | Wirkstoff |
| PVA 4-88 | 51 | Polymer |
| PVA 40-88 | 16 | Polymer |
| Xylitol | 2 | Süßstoff und Zerfallsbeschleuniger |
| Natriumbisulfit | 0,1 | Antioxidationsmittel |
| Tween 80 | 1 | Weichmacher |
| Sorbitol | 9 | Süßstoff und Zerfallsbeschleuniger |
| Sucralose | 1 | Süßstoff, Zerfallsbeschleuniger und Feuchthaltemittel |
| Glycerol | 9,9 | Weichmacher |
| Flächengewicht | 176 g/m² | |
| Lösemittel | Wasser | |
| Zerfallszeit | 5 s | |

**Formulierung C4: PVA-Schaumformulierung; PVA-Lösungen wurden auf pH 4 eingestellt**

| Ausgangsstoffe | Zusammensetzung [Gew.-%] | Art / Funktion |
|---|---|---|
| Naloxon HCl Dihydrat | 10 | Wirkstoff |
| PVA 4-88 | 51 | Polymer |
| PVA 40-88 | 16 | Polymer |
| Xylitol | 2 | Süßstoff und Zerfallsbeschleuniger |
| Natriumbisulfit | 0,1 | Antioxidationsmittel |
| Tween 80 | 1 | Weichmacher |
| Sorbitol | 9 | Süßstoff und Zerfallsbeschleuniger |
| Sucralose | 1 | Süßstoff, Zerfallsbeschleuniger und Feuchthaltemittel |
| Glycerol | 9,9 | Weichmacher |
| Flächengewicht | 152 g/m² | |
| Lösemittel | Wasser | |
| Zerfallszeit | 4 s | |

**Formulierung C5: PVA-Schaumformulierung; PVA-Lösungen wurden auf pH 8 eingestellt**

| Ausgangsstoffe | Zusammensetzung [Gew.-%] | Art / Funktion |
|---|---|---|
| Naloxon HCl Dihydrat | 10 | Wirkstoff |
| PVA 4-88 | 51 | Polymer |
| PVA 40-88 | 16 | Polymer |
| Xylitol | 2 | Süßstoff und Zerfallsbeschleuniger |
| Natriumbisulfit | 0,1 | Antioxidationsmittel |
| Tween 80 | 1 | Weichmacher |
| Sorbitol | 9 | Süßstoff und Zerfallsbeschleuniger |
| Sucralose | 1 | Süßstoff, Zerfallsbeschleuniger und Feuchthaltemittel |
| Glycerol | 9,9 | Weichmacher |
| Flächengewicht | 153 g/m² | |
| Lösemittel | Wasser | |
| Zerfallszeit | 7 s | |

**Formulierung C6: PVA-Filmformulierung**

| Ausgangsstoffe | Zusammensetzung [Gew.-%] | Art / Funktion |
|---|---|---|
| Naloxon HCl Dihydrat | 10 | Wirkstoff |
| PVA 4-88 | 56 | Polymer |
| PVA 40-88 | 6 | Polymer |
| Xylitol | 2 | Süßstoff und Zerfallsbeschleuniger |
| Span 83 | 1 | Emulgator |
| Natriumbisulfit | 0,1 | Antioxidationsmittel |
| Tween 80 | 1 | Weichmacher |
| Sorbitol | 9 | Süßstoff und Zerfallsbeschleuniger |
| Sucralose | 1 | Süßstoff, Zerfallsbeschleuniger und Feuchthaltemittel |
| Glycerol | 8,9 | Weichmacher |
| Reisstärke | 5 | Sprengmittel |
| Flächengewicht | 147 g/m² | |
| Lösemittel | Wasser | |
| Zerfallszeit | 20 s | |

**Formulierung C7: PVA-Filmformulierung; PVA-Lösungen wurden auf pH 4 eingestellt**

| Ausgangsstoffe | Zusammensetzung [Gew.-%] | Art / Funktion |
|---|---|---|
| Naloxon HCl Dihydrat | 10 | Wirkstoff |
| PVA 4-88 | 56 | Polymer |
| PVA 40-88 | 6 | Polymer |
| Xylitol | 2 | Süßstoff und Zerfallsbeschleuniger |
| Span 83 | 1 | Emulgator |
| Natriumbisulfit | 0,1 | Antioxidationsmittel |
| Tween 80 | 1 | Weichmacher |
| Sorbitol | 9 | Süßstoff und Zerfallsbeschleuniger |
| Sucralose | 1 | Süßstoff, Zerfallsbeschleuniger und Feuchthaltemittel |
| Glycerol | 8,9 | Weichmacher |
| Reisstärke | 5 | Sprengmittel |
| Flächengewicht | 157 g/m² | |
| Lösemittel | Wasser | |
| Zerfallszeit | 22 s | |

**Formulierung C8: PVA-Filmformulierung; PVA-Lösungen wurden auf pH 8 eingestellt**

| Ausgangsstoffe | Zusammensetzung [Gew.-%] | Art / Funktion |
|---|---|---|
| Naloxon HCl Dihydrat | 10 | Wirkstoff |
| PVA 4-88 | 56 | Polymer |
| PVA 40-88 | 6 | Polymer |
| Xylitol | 2 | Süßstoff und Zerfallsbeschleuniger |
| Span 83 | 1 | Emulgator |
| Natriumbisulfit | 0,1 | Antioxidationsmittel |
| Tween 80 | 1 | Weichmacher |
| Sorbitol | 9 | Süßstoff und Zerfallsbeschleuniger |
| Sucralose | 1 | Süßstoff, Zerfallsbeschleuniger und Feuchthaltemittel |
| Glycerol | 8,9 | Weichmacher |
| Reisstärke | 5 | Sprengmittel |
| Flächengewicht | 154 g/m² | |
| Lösemittel | Wasser | |
| Zerfallszeit | 28 s | |

**Formulierung C9: PVA-Schaumformulierung**

| Ausgangsstoffe | Zusammensetzung [Gew.-%] | Art / Funktion |
|---|---|---|
| Nalmefen HCl | 10 | Wirkstoff |
| PVA 4-88 | 51 | Polymer |
| PVA 40-88 | 16 | Polymer |
| Xylitol | 2 | Süßstoff und Zerfallsbeschleuniger |
| Tween 80 | 1 | Weichmacher |
| Sorbitol | 9 | Süßstoff und Zerfallsbeschleuniger |
| Sucralose | 1 | Süßstoff, Zerfallsbeschleuniger und Feuchthaltemittel |
| Glycerol | 10 | Weichmacher |
| Flächengewicht | 164 g/m² | |
| Lösemittel | Wasser | |
| Zerfallszeit | 10 s | |

### Beispiel 3:

Es wurden orale Dünnfilme mit Vortioxetin HBr als Wirkstoff hergestellt und deren Zerfallszeiten nach Methode Ph. Eur. 2.9.1. mit Gewicht und Platte bestimmt.

**Formulierung D1: PVA-Schaumformulierung**

| Ausgangsstoffe | Zusammensetzung [Gew.-%] | Art / Funktion |
|---|---|---|
| Vortioxetin HBr | 30 | Wirkstoff |
| PVA 4-88 | 36 | Polymer |
| PVA 40-88 | 11 | Polymer |
| Xylitol | 2 | Süßstoff und Zerfallsbeschleuniger |
| Natriumbisulfit | | Antioxidationsmittel |
| Tween 80 | 1 | Weichmacher |
| Sorbitol | 9 | Süßstoff und Zerfallsbeschleuniger |
| Sucralose | 1 | Süßstoff, Zerfallsbeschleuniger und Feuchthaltemittel |
| Glycerol | 10 | Weichmacher |
| Flächengewicht | 167,5 g/m² | |
| Lösemittel | Wasser | |
| Zerfallszeit | 5 s | |

**Formulierung D2: PVA-Filmformulierung**

| Ausgangsstoffe | Zusammensetzung [Gew.-%] | Art / Funktion |
|---|---|---|
| Vortioxetin HBr | 30 | Wirkstoff |
| PVA 4-88 | 37 | Polymer |
| PVA 40-88 | 4 | Polymer |
| Xylitol | 2 | Süßstoff und Zerfallsbeschleuniger |
| Span 83 | 1 | |
| Tween 80 | 1 | Weichmacher |
| Sorbitol | 9 | Süßstoff und Zerfallsbeschleuniger |
| Sucralose | 1 | Süßstoff, Zerfallsbeschleuniger und Feuchthaltemittel |
| Glycerol | 9 | Weichmacher |
| Reisstärke | 6 | Sprengmittel |
| Flächengewicht | 153,6 g/m² | |
| Lösemittel | Wasser | |
| Zerfallszeit | 19 s | |

**Formulierung D3: PVA-Filmformulierung**

| Ausgangsstoffe | Zusammensetzung [Gew.-%] | Art / Funktion |
|---|---|---|
| Vortioxetin HBr | 30 | Wirkstoff |
| PVA 4-88 | 36 | Polymer |
| PVA 40-88 | 11 | Polymer |
| Xylitol | 2 | Süßstoff und Zerfallsbeschleuniger |
| Tween 80 | 1 | Weichmacher |
| Sorbitol | 9 | Süßstoff und Zerfallsbeschleuniger |
| Sucralose | 1 | Süßstoff, Zerfallsbeschleuniger und Feuchthaltemittel |
| Glycerol | 7 | Weichmacher |
| Reisstärke | 3 | Sprengmittel |
| Flächengewicht | 148,9 g/m² | |
| Lösemittel | Wasser | |
| Zerfallszeit | 4 s | |

Für die obigen Formulierungen D1 bis D3 wurde die *in* vitro-Permeation mittels Franz-Diffusionszellen (Volumen 10 mL) bei 37°C durchgeführt. Zu vorbestimmten Wechselzeiten wurde das jeweils verwendete Akzeptormedium komplett durch ein Neues ausgetauscht und der Gehalt an permeierter Wirkstoffmenge in diesen Akzeptorlösungen mittels HPLC bestimmt.

Als als Akzeptormedium wurde Phosphatpuffer pH 7,4 eingesetzt.

Als Hautmodell wurde dermatomisierte Haut aus der Speiseröhre eines Schweins mit einer Schichtdicke von 400µm verwendet.

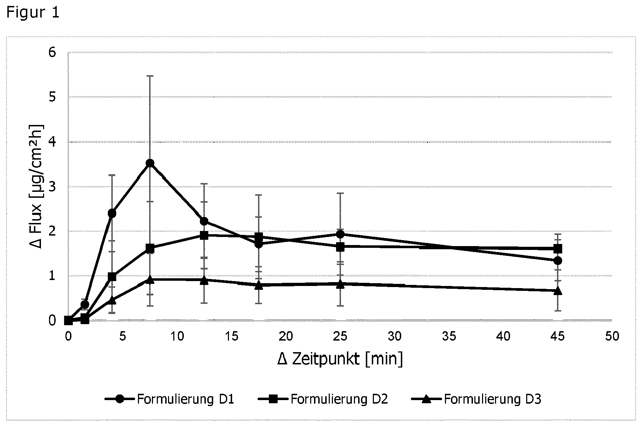

Die Ergebnisse sind in Figur 1 zusammengefasst.

## Patentansprüche

1. Oraler dünner Film, umfassend mindestens eine Matrixschicht, wobei die mindestens eine Matrixschicht mindestens einen Polyvinylalkohol mit einem niedrigeren mittleren Molekulargewicht von 10.000 bis etwa 75.000 g/mol und mindestens einen Polyvinylalkohol mit einem höheren mittleren Molekulargewicht von 140.000 bis 300.000 g/mol und mindestens einen pharmazeutischen Wirkstoff umfasst, wobei der mindestens eine Polyvinylalkohol mit einem niedrigeren mittleren Molekulargewicht und der mindestens eine Polyvinylalkohol mit einem höheren mittleren Molekulargewicht in einer Gesamtmenge von 15 bis 70 Gew.-%, bezogen auf das Gesamtgewicht der Matrixschicht, in der Matrixschicht vorhanden sind und das Gewichtsverhältnis von dem mindestens eine Polyvinylalkohol mit einem niedrigeren mittleren Molekulargewicht zu dem mindestens einen Polyvinylalkohol mit einem höheren mittleren Molekulargewicht im Bereich von 20:1 bis 2:1 liegt.

2. Oraler dünner Film nach Anspruch 1, wobei der mindestens eine Polyvinylalkohol mit einem niedrigeren mittleren Molekulargewicht ein mittleres Molekulargewicht im Bereich von 10.000 bis etwa 40.000 g/mol, bevorzugt 25.000 bis 35.000 g/mol, aufweist und/oder der mindestens eine Polyvinylalkohol mit einem höheren mittleren Molekulargewicht ein mittleres Molekulargewicht im Bereich von 140.000 bis 220.000 g/mol, bevorzugt 200.000 bis 210.000 g/mol aufweist.

3. Oraler dünner Film nach einem der vorhergehenden Ansprüche, wobei der mindestens eine Polyvinylalkohol mit einem niedrigeren mittleren Molekulargewicht und der mindestens eine Polyvinylalkohol einem höheren mittleren Molekulargewicht in einer Gesamtmenge von 20 bis 55 Gew.-%, insbesondere 25 bis 40 Gew.-%, bezogen auf das Gesamtgewicht der Matrixschicht, in der Matrixschicht vorhanden sind.

4. Oraler dünner Film nach einem der vorhergehenden Ansprüche, wobei das Gewichtsverhältnis von dem mindestens einen Polyvinylalkohol mit einem niedrigeren mittleren Molekulargewicht zu dem mindestens einen Polyvinylalkohol mit einem höheren mittleren Molekulargewicht im Bereich von 15:1 bis 3:1, bevorzugt von 10:1 bis 2:1, insbesondere von 9:1 bis 7:1, liegt.

5. Oraler dünner Film nach einem der vorhergehenden Ansprüche, wobei die Menge des mindestens einen Polyvinylalkohol mit einem höheren mittleren Molekulargewicht in der Matrixschicht im Bereich von 1 bis 18 Gew.-%, vorzugsweise von 1 bis 12 Gew.-%, besonders bevorzugt von 2 bis 10 Gew.-%, insbesondere von 3 bis 4 Gew.-%, bezogen auf das Gesamtgewicht der Matrixschicht, liegt.

6. Oraler dünner Film nach einem der vorhergehenden Ansprüche, wobei der mindestens eine Polyvinylalkohol mit einem niedrigeren mittleren Molekulargewicht Polyvinylalkohol 4-88 ist und/oder der mindestens eine Polyvinylalkohol mit einem höheren mittleren Molekulargewicht Polyvinylalkohol 40-88 ist.

7. Oraler dünner Film nach einem der vorhergehenden Ansprüche, wobei die Matrixschicht ferner ein mindestens ein hygroskopisches Additiv mit mehr als 3 Kohlenstoffatomen aufweist, welches bevorzugt ausgewählt ist aus Zuckern, Zuckeralkoholen, Stärken und Stärkederivaten oder einer Mischung davon.

8. Oraler dünner Film nach Anspruch 7, wobei das mindestens eine hygroskopische Additiv mit mehr als 3 Kohlenstoffatomen ausgewählt ist aus Sorbitol, Mannit (Mannitol), Isomalt, Lactit, Sorbit (Sorbitol oder Glucitol) und Xylit (Xylitol), Threit, Erythrit, Stärken, einschließlich Stärkederivaten, Sucralose oder einer Mischung von zwei oder mehr dieser Verbindungen.

9. Oraler dünner Film nach Anspruch 7 oder Anspruch 8, wobei das mindestens eine hygroskopische Additiv mit mehr als 3 Kohlenstoffatomen in einer Menge von 5 bis 20 Gew.-%, bevorzugt 5 bis 15 Gew.-%, bezogen auf das Gesamtgewicht der Matrixschicht, vorhanden ist.

10. Oraler dünner Film nach einem der vorhergehenden Ansprüche, wobei der mindestens eine pharmazeutische Wirkstoff Ulipristalacetat, bevorzugt mikronisiertes Ulipristalacetat, oder Ketamin, vorzugsweise (S)-Ketamin, , oder Naloxon, oder Nalmefen oder Vortioxetin, oder ein jeweiliges pharmazeutisch akzeptables Salz davon umfasst oder ist, wobei Ulipristalacetat oder Ketamin besonders bevorzugt sind.

11. Oraler dünner Film nach einem der vorhergehenden Ansprüche, wobei der mindestens eine pharmazeutische Wirkstoffoder ein pharmazeutisch akzeptables Salz davon in einer Menge von 10 bis 60 Gew.-%, bevorzugt 20 bis 55 Gew.-%, bevorzugter 30 bis 50 Gew.-%, bezogen auf das Gesamtgewicht der Matrixschicht, in der Matrixschicht vorhanden ist.

12. Oraler dünner Film nach einem der vorhergehenden Ansprüche, wobei die Matrixschicht ferner mindestens einen Hilfsstoff ausgewählt aus der Gruppe, bestehend aus Farbstoffen, Aromastoffen, Süßstoffen, Weichmachern, geschmacksmaskierenden Mitteln, Emulgatoren, Enhancern, Sprengmitteln, Lösungsvermittlern, pH-Regulatoren, Konservierungsmitteln und/oder Antioxidationsmitteln, umfasst, wobei die Matrixschicht bevorzugt einen oder mehrere Weichmacher, vorzugsweise Glycerin, umfasst.

13. Oraler dünner Film nach einem der vorhergehenden Ansprüche, wobei die Matrixschicht eine nicht geschäumte Matrixschicht oder eine geschäumte Matrixschicht ist, wobei die Matrixschicht bevorzugt ein Schaum ist.

14. Oraler dünner Film nach einem der vorhergehenden Ansprüche, wobei das Flächengewicht der Matrixschicht 50 bis 220 g/m², bevorzugt 50 bis 150 g/m², besonders bevorzugt 70 bis 130 g/m², beträgt.

15. Oraler dünner Film nach einem der vorhergehenden Ansprüche zur Verwendung als Arzneimittel, bevorzugt als Notfallkontrazeptivum oder bei der Behandlung oder Prävention von Schmerzen und/oder Depressionen oder als Anästhetikum und/oder in der Behandlung von Opiatüberdosierung.
